# EUROPEAN PATENT SPECIFICATION

(11) **EP 2 956 122 B1**
(45) Date of publication and mention of the grant of the patent: **27.01.2021**
(21) Application number: 14752091.0
(22) Date of filing: 18.02.2014
(51) Int. Cl.: A61K 31/713, A61K 9/00, A61K 41/00, A61K 9/51

(54) **SUPRAMOLECULAR MAGNETIC NANOPARTICLES**
SUPRAMOLEKULARE MAGNETISCHE NANOPARTIKEL
NANOPARTICULES MAGNÉTIQUES SUPRAMOLÉCULAIRES

(30) Priority: 15.02.2013 US 201361765350 P
(43) Date of publication of application: 23.12.2015
(73) Proprietor: The Regents of the University of California, Oakland, CA 94607-5200 (US); Office of Research Affairs Uif Yonsei University, Seoul 120-749 (KR)
(72) Inventor: CHEON, Jinwoo, Seoul 120-749 (KR); NOH, Seung-hyun, Seoul 120-749 (KR); TSENG, Hsian-Rong, Los Angeles, CA 90025 (US); CHEN, Kuan-Ju, Cambridge, MA 02139 (US)
(74) Representative: Rambelli, Paolo
(86) International application number: PCT/US2014/016903
(87) International publication number: WO 2014/127357

(56) References cited:
- US-A1- 2004 109 888
- US-A1- 2007 117 177
- US-A1- 2011 305 685
- US-A1- 2011 305 685
- CHARLES SANSON ET AL: "Doxorubicin Loaded Magnetic Polymersomes: Theranostic Nanocarriers for MR Imaging and Magneto-Chemotherapy", ACS NANO, vol. 5, no. 2, 22 February 2011 (2011-02-22), pages 1122-1140, XP055301307, US ISSN: 1936-0851, DOI: 10.1021/nn102762f
- Kuan-Ju Chen ET AL: "Supramolecular Nanoparticles for Molecular Diagnostics and Therapeutics" In: "Supramolecular Chemistry", 15 March 2012 (2012-03-15), John Wiley & Sons, Ltd, Chichester, UK, XP055300977, ISBN: 978-0-470-66134-5 DOI: 10.1002/9780470661345.smc194, * figures 1-3 * * page 3, left-hand column, paragraph 2 - page 6, left-hand column, paragraph 1; figure 10 *
- CHEN, K.-J. ET AL.: 'The therapeutic efficacy of camptothecin- encapsulated supramolecular nanoparticles' BIOMATERIALS vol. 33, 2012, pages 1162 - 1169, XP028115493
- PARK, I.-K. ET AL.: 'Supramolecular assembly of cyclodextrin-based nanoparticles on solid surfaces for gene delivery' LANGMUIR vol. 22, 2006, pages 8478 - 8484, XP055087466
- LEE, J.-H. ET AL.: 'On-demand drug release system for in vivo cancer treatment through self-assembled magnetic nanoparticles' ANGEWANDTE CHEMIE INTERNATIONAL EDITION vol. 125, 20 March 2013, pages 4480 - 4484, XP055277766

## Description

This application claims priority to provisional US application no. 61,765,350, filed February 15, 2013.

### STATEMENT OF GOVERNMENT SUPPORT

This invention was made with Government support under Grant No. R21 GM098982, awarded by the Department of Health and Human Services, The National Institutes of Health (NIH). The Government has certain rights in the invention.

### TECHNICAL FIELD

Herein disclosed are supramolecular magnetic nanoparticles, use of supramolecular magnetic nanoparticles for on-demand drug release, and methods of making and using the same.

### BACKGROUND

Nanoparticle therapeutics are typically particles comprised of therapeutic entities, such as small-molecule drugs, peptides, proteins and nucleic acids, and components that assemble with the therapeutic entities, such as lipids and polymers. Such nanoparticles can have enhanced anticancer effects compared with the therapeutic entities they contain. This is owing to more specific targeting to tumor tissues via improved pharmacokinetics and pharmacodynamics, as well as active intracellular delivery. These properties depend on the size and surface properties (including the presence of targeting ligands) of the nanoparticles.

### SUMMARY

The subject matter of the invention is defined by the appended claims. Any subject-matter falling outside the scope of the claims is provided for information purposes only.

Disclosed is a supramolecular magnetic nanoparticle (SMNP) including a plurality of structural components each including a plurality of binding elements; at least one magnetic nanoparticle each including a plurality of binding elements; a plurality of binding components each including a plurality of binding regions, where each of the binding regions is adapted to bind to a binding element; a plurality of terminating components each including a terminating element , where the terminating element is adapted to occupy a binding region; and a cargo; where the plurality of terminating components are present in a sufficient quantity relative to the plurality of binding regions of the plurality of binding components to terminate further binding.

Also disclosed is a method of delivering a drug including administering a supramolecular magnetic nanoparticle (SMNP) as described above to a subject, and applying an alternating magnetic field (AMF) to the SMNP within the subject.

The herein disclosed method of making a supramolecular magnetic nanoparticle (SMNP), includes combining: a plurality of structural components each including a plurality of binding elements; at least one magnetic nanoparticle each including a plurality of binding elements; a plurality of binding components each including a plurality of binding regions, where each of the binding regions is adapted to bind to a binding element; a plurality of terminating components each including a terminating element , where the terminating element is adapted to occupy a binding region; and a cargo; where a ratio of an amount of structural components to magnetic nanoparticles to binding components to terminating components are selected in accordance with a predetermined size of said SMNPs, and where the structural components, magnetic nanoparticles, binding components, and terminating components self-assemble into SMNPs having substantially the predetermined size.

Other features, objects and embodiments will be apparent from the description, claims and drawings.

### BRIEF DESCRIPTION OF THE DRAWINGS

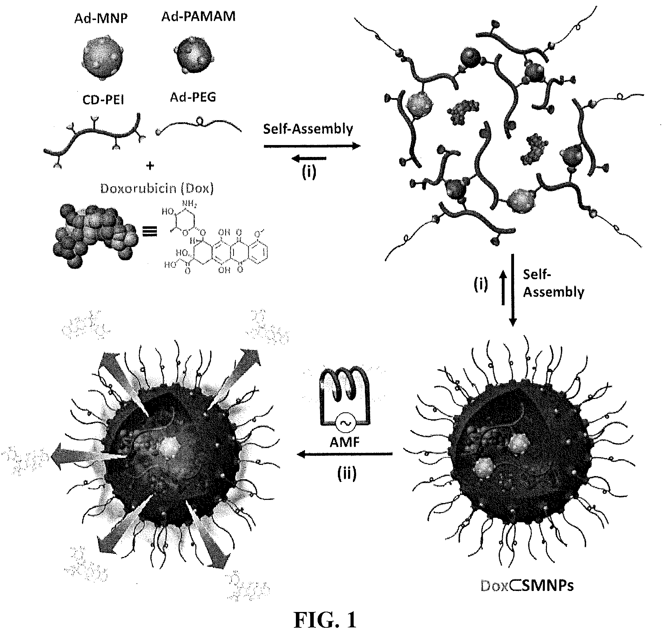

FIG. 1 is a schematic illustration summarizing the molecular design, self-assembly and function of magnetothermally responsive doxorubicin (**Dox**)-encapsulated supramolecular magnetic nanoparticles **(DoxcSMNPs).** i) The self-assembled synthetic strategy is employed for the preparation of **DoxcSMNPs,** which is made from a fluorescent anti-cancer drug **(Dox)** and four molecular building blocks: **Ad-PAMAM,** 6-nm **Ad**-grafted Zn_{0.4}Fe_{2.6}O₄ superparamagnetic nanoparticle **(Ad-MNP), CD-PEI,** and **Ad-PEG.** ii) The embedded **Ad-MNP** serves as a built-in heat transformer that triggers the burst release of **Dox** molecules from the magnetothermally responsive **SMNP** vector, achieving on-demand drug release upon the remote application of a time-varying magnetic field, also called an alternating magnetic field (AMF).

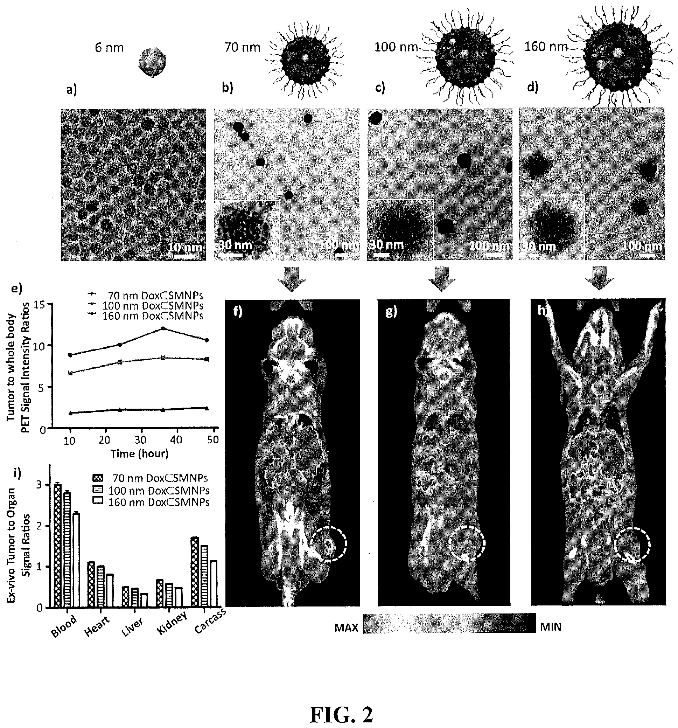

FIGS. 2a-2i illustrate characterization and biodistribution of **DoxcSMNPs.** a-d) Transmission electron microscope (TEM) images of a) 6-nm **Ad-MNP** and **DoxcSMNPs** with various sizes of b) 70 ± 9, c) 96 ± 7, and d) 161 ± 8 nm. Insets: Higher magnification TEM images of each **DoxcSMNPs.** e) Time dependent accumulation of the three **DoxcSMNPs** in the tumor versus whole body of NU/NU mice measured by micro-PET. f-h) Two-dimensional cross-sections of static filtered back-projection micro-PET images of mice bearing DLD-1 tumor at 36 h post-injection of 70-nm, 100-nm, and 160-nm **⁶⁴Cu-labeled DoxcSMNPs.** The 70-nm **DoxcSMNPs** showed the highest tumor-specific uptake among the three studies. i) Tumor to organ signal ratios quantified from *ex vivo* biodistribution data of mice treated with 70-nm, 100-nm, and 160-nm **DoxcSMNPs** at the termination of the study (48 h post intravenous injection).

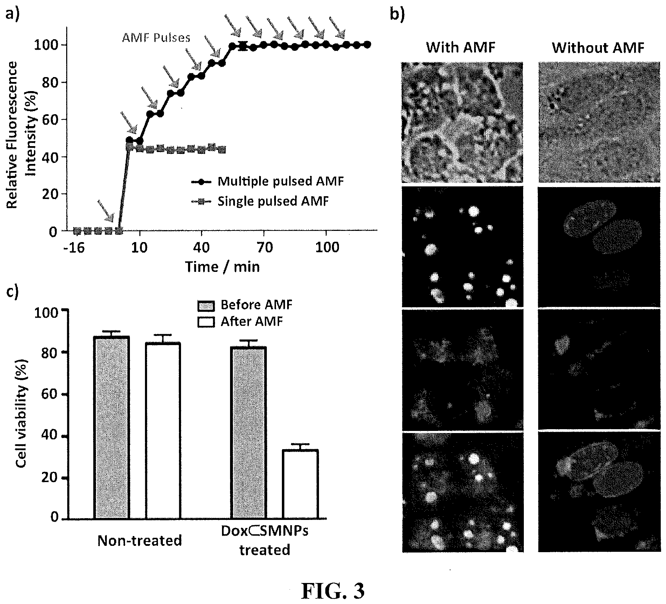

FIG. 3. illustrates in vitro drug release and therapeutic efficacy of 70-nm **DoxcSMNPs.** a) **Dox** release profiles upon the application of AMF in either multiple pulses (black line; 2 min of pulse duration with 8 min of non-pulsed intermittence) or as a single pulse (red line; 2 min of pulse duration), *ca.* 50% of encapsulated **Dox** molecules release in the first AMF pulse and the rest of **Dox** releases stepwise in subsequent AMF pulses. b) Fluorescence microscope images of DLD-1 colon cancer cells treated with **DoxcSMNPs** with (left) and without (right) the application of a 10-min continuous AMF (500 kHz, 37.4 kA/m). Images from top to bottom: Differential interference contrast (DIC) image showing the cell morphology; DAPI stained image showing the nuclei; **Dox** fluorescence indicating the presence of **Dox;** merged image of DAPI and **Dox. Dox** molecules were burst-released from **DoxcSMNPs** upon AMF application and then entered into nuclei, which led to cell apoptosis. c) Cell viability results of DLD-1 cells treated with and without **DoxcSMNPs** before and after application of AMF for 10 min via CCK-8 assay. The viability of **DoxcSMNPs** treated cells is decreased to *ca.* 30% after the application of AMF.

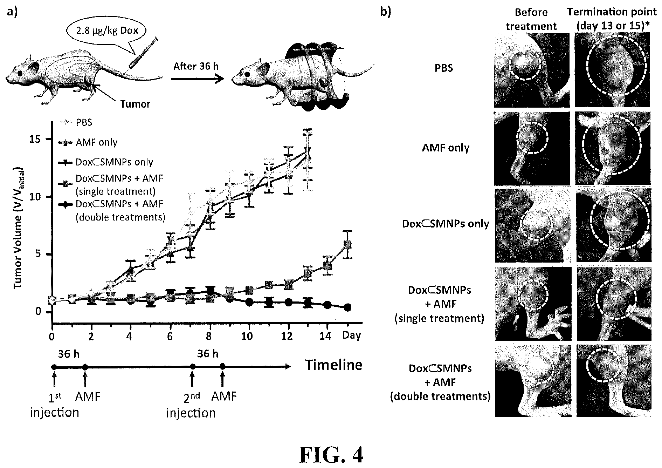

FIG. 4 illustrates the evaluation of in vivo therapeutic efficacy. a) Treatment scheme of **DoxcSMNPs** in mouse and results of tumor volume change over the course of treatment (15 days) in DLD-1 xenografted mice (n=3) treated with **DoxcSMNPs** (w/ and w/o application of AMF), and other controls (AMF only and PBS only). All injections were done on day 0 (and day 7 for the double injection group) when the tumor volume reached 100 mm³; AMF application was performed at 36 h post-injection. The best tumor suppression result was observed in the group treated with a double injection of **Dox⊂SMNPs** with AMF application. The group treated with single injection of **DoxcSMNPs** with AMF, and the other control groups (i.e., treated with **DoxcSMNPs** only, AMF only and PBS) show either lesser degree or none of tumor suppression effects. b) Tumor images of groups treated with **DoxcSMNPs** w/ and w/o application of AMF and other controls, before treatment (left panels) and at the termination point (right panels). The termination point of the experiment occurred either on day 15 or when the tumor volume reached 1500 mm³.

### DETAILED DESCRIPTION

Embodiments of the invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. A person skilled in the relevant art will recognize that other components and configurations can be used without departing from the scope of the appended claims.

The intrinsic nature of small-molecule chemotherapeutics, including i) limited aqueous solubility, ii) systemic toxicity due to non-specific whole-body distribution, and iii) potential development of drug resistance after initial administration, compromises their treatment efficacy.⁽¹⁾ Nanoparticle (NP)-based drug delivery systems offer a promising solution to overcome these intrinsic limitations and begin to revolutionize the disease management in clinical oncology.⁽²⁾ For instance, the intraparticular space of a NP vector can be employed to package drug payloads without constrain associated with their solubility. Further, NP vectors exhibit enhanced permeability and retention (EPR) effects⁽³⁾ that facilitate the differential uptake, leading to preferential spatio-distribution in tumor.⁽⁴⁾ However, conventional NP drug delivery systems tend to passively release drug payloads depending on the tumor microenvironment, which limits the ability to release an effective drug concentration at a desired time point. Therefore, it is important to engineer a stimuli-responsive drug release mechanism into a NP-based delivery system with a goal of achieving spatio-temporal control by which an acute level of drug concentration can be delivered at the time point the NP vectors reach maximum tumor accumulation.⁽⁵⁾ By doing so, it is expected to dramatically improve therapeutic effects in tumor and effectively reduce systematic toxicity by using a minute amount of drugs.⁽⁶⁾

Disclosed are supramolecular magnetic nanoparticles structures, also called supramolecular nanoparticles (SMNPs), having a plurality of structural components each including a plurality of binding elements; at least one magnetic nanoparticle each including a plurality of binding elements; a plurality of binding components each including a plurality of binding regions, wherein each of the binding regions is adapted to bind to a binding element; a plurality of terminating components each including a terminating element , wherein the terminating element is adapted to occupy a binding region; and a cargo.

Supramolecular nanoparticles structures are described in WO 2010/099466, corresponding to US 2011/030585, that also discloses therapeutical uses thereof. US 2011/305685 discloses supramolecular magnetic nanoparticles (SMNPs) as structural components with binding elements for bio-distribution.

Charles Sanson et al., ACS NANO, Vol. 5, No. 2, 22 February 2011, pages 1122-1140, entitled "Doxorubicin Loaded Magnetic Polymersomes: Theranostic Nanocarriers for MR Imaging and Magneto-Chemotherapy" discloses hydrophobically modified maghemite nanoparticles encapsulated within the membrane of poly(trimethylene carbonate)-b-poly(L-glutamic) acid block copolymer vescicles, using a nanoprecipitation process. The formation method gives access to highly magnetic nanoparticles (loaded up to 70 wt%) with good control over the vesicles size. The simultaneous loading of maghemite nanoparticles and doxorubicin was also achieved by nanoprecipitation.

The structural components, magnetic nanoparticle, and binding components self-assemble when brought into contact to form a supramolecular magnetic nanoparticle (SMNP). The terminating elements of the terminating components occupy binding regions of the binding components to terminate further binding when the terminating components are present in a sufficient quantity relative to the binding regions of the binding components. Presence of a sufficient quantity of a terminating components relative to the binding regions of the binding components allows the formation of a discrete components self-assemble when brought into contact to form a supramolecular having a predetermined particle size, rather than an extended network of indeterminate size. The cargo can be incorporated in the SMNP during self-assembly.

The binding elements of the structural component bind to binding regions of the binding component. As the structural component includes a plurality of binding elements, it may bind to multiple binding regions of a single binding component, may bind to multiple binding components, or both. In general, binding elements of structural components bind to multiple binding regions on multiple binding components, undergoing spontaneous self-assembly, forming a crosslinked network or hydrogel between structural components and binding components. The terminating elements of terminating components also bind to binding regions of the binding component. Generally, a single binding region binds to only one binding element or one terminating element at a time. In this way, when the terminating component is present in sufficient concentration relative to the binding regions of the binding components, the terminating component competes for binding regions on the binding component, thereby constraining the continuous propagation of the crosslinked network. In this way, the terminating component terminates growth of the crosslinked network and a discrete particle is formed.

The structural component, binding component, magnetic nanoparticle, and terminating components bind to each other by one or more intermolecular forces. Examples of intermolecular forces include hydrophobic interactions, biomolecular interactions, hydrogen bonding interactions, π-π interactions, electrostatic interactions, dipole-dipole interactions, or van der Waals forces. Examples of biomolecular interactions include DNA hybridization, a protein-small molecule interaction (e.g. protein-substrate interaction (e.g. a streptavidin-biotin interaction) or protein-inhibitor interaction), an antibody-antigen interaction or a protein-protein interaction. Examples of other interactions include inclusion complexes or inclusion compounds, e.g. adamantane-β-cyclodextrin complexes or diazobenzene-α-cyclodextrin complexes. Generally, the intermolecular forces binding the components of the SMNP structure are not covalent bonds.

### Structural Component

The disclosed structural component has a plurality of binding elements that bind to the binding regions of the binding components. The binding element is a chemical moiety that binds to the binding region of the binding component by one or more intermolecular forces. The binding element of the structural component and the binding region of the binding element are specifically selected to bind to each other, and may use molecular recognition properties to identify the binding regions.

The disclosed structural component is at least one of an inorganic or organic core.

Inorganic cores include inorganic nanoparticles, such as metal nanoparticles (e.g. gold nanoparticles, silver nanoparticles, silicon nanoparticles, or other metals). Other inorganic nanoparticles include metal oxide nanoparticles (e.g. silica nanoparticles or iron oxide nanoparticles, including doped iron oxide nanoparticles), and nanoparticles of other inorganic compounds. Functional nanoparticles may be used, such as, magnetic nanoparticles, quantum dots (e.g., CdS or CdSe nanoparticles), or semiconductive oxide particles.

In some embodiments, the SMNP includes both an organic core and an inorganic core including a magnetic nanoparticle. The organic core can serve as a structural component, and the inorganic core can serve as the magnetic nanoparticle. In some embodiments, the inorganic core serves as both a structural component and magnetic nanoparticle.

A class of magnetic nanoparticles is described in, e.g., U.S. Patent Application Publication No. 2013/0046274.

In some embodiments, the inorganic core is spherical. In other embodiments, the morphology of the inorganic core may be triangular, cubic, star-like, rod-like, shell, diamond-like, plate-like, pyramidal, irregular or cage structure.

In some embodiments, the inorganic core has a maximum dimension of less than about 100 nm. The maximum dimension of the inorganic core may be less than about 70nm, less than about 50nm, less than about 25 nm, less than about 10 nm, or less than about 5 nm. The size of the inorganic core may vary based on the function of the supramolecular structure.

Where a range of values is provided in the present application, it is understood that each intervening value, to the tenth of the unit of the lower limit unless the context clearly dictates otherwise, between the upper and lower limit of that range and any other stated or intervening value in that stated range, is encompassed within the invention. The end values of any range are included in the range.

Numerous inorganic nanoparticles are known in the art. The inorganic core binds to binding regions of the binding component. In some embodiments, the binding component has binding regions that bind to the inorganic core directly. In other embodiments, the surface of the inorganic core is derivatized with a plurality of binding elements that bind to the binding regions of the binding component by one or more intermolecular forces.

In some embodiments, a plurality of inorganic core particles are present in the SMNP. In such cases, the plurality of inorganic core particles bind with a plurality of binding components to form a crosslinked network or hydrogel. The continuous propagation of the crosslinked network is constrained or terminated by terminating components that also bind to the binding regions of the binding component. In some embodiments, a plurality of inorganic cores and a plurality of organic cores are present in the SMNP, and both are integrated in the crosslinked network or hydrogel.

The disclosed structural component may be an organic core. Organic cores include dendrimers, polymers, proteins, oligosaccharides, micelles, liposomes or vesicles. In some embodiments, the organic core is a dendrimer, polymer or polypeptide. In some embodiments, the structural component is a dendrimer (e.g. polyamidoamine dendrimer or PAMAM), branched polyethyleneimide (PEI), linear polyethyleneimide, polylysine, polylactide, polylactide-co-glycolide, polyanhydrides, poly-ε-caprolactones, polymethyl methacrylate, poly(N-isopropyl acrylamide) or polypeptide. In some embodiments, the organic core is a polyamidoamine dendrimer. In some embodiments, the organic core is a poly-L-lysine polymer.

In some embodiments, the binding regions of the binding component bind to binding elements present as part of the organic core structure. In other embodiments, the organic core is derivatized with a plurality of binding elements. The binding elements bind to the binding regions of the binding component by one or more intermolecular forces and self-assemble into a crosslinked network or hydrogel. The continuous propagation of the crosslinked network is constrained or terminated by terminating components that also bind to the binding regions of the binding component. The binding elements and binding regions may be selected based on the type of binding desired and may use molecular recognition properties in some embodiments.

Numerous dendrimers are known in the art. The advantage of dendrimer cores lies in their rapid synthesis, and easy ability to be functionalized with binding elements. The dendrimer may be synthesized to include binding elements as part of the structure. Alternatively, during dendrimer synthesis, a reactive functionality is present at each terminating point, which may be terminated with a chemical moiety that functions as a binding element to bind to the binding region of the binding component. Examples of specific dendrimers include polyamidoamine dendrimer or PAMAM.

Numerous polymers are known in the art. The advantage of polymer cores lies in their rapid synthesis and ability to be easily functionalized with binding elements. The polymer may be synthesized to include binding elements as part of the structure. Alternatively, reactive functional groups on a polymer may be derivatized with a chemical moiety that functions as a binding element. For example, polypeptides having lysine residues have reactive amine (-NH2) groups which may be functionalized with binding elements. A specific example is poly-L-lysine.

In some embodiments, two or more different structural components are present, so long as both have binding elements that bind to binding regions of the binding components.

In some embodiments, the structural component comprises a polyamidoamine dendrimer derivatized with a binding element, such as adamantane. In other embodiments, the structural component is an inorganic nanoparticle (for example, a magnetic nanoparticle such as a doped iron oxide nanoparticle) derivatized with adamantane.

### Terminating Component

The terminating components include terminating elements adapted to occupy binding regions of the binding components. This acts to constrain the continuous propagation of the crosslinked network when the terminating components are present in a sufficient quantity relative to the binding regions of the binding components. The structural component, magnetic nanoparticle, and binding component self-assemble into a supramolecular structure, while the terminating components occupy binding regions and prevent further self-assembly between the structural component, magnetic nanoparticle, and binding component. The extent to which the terminating component limits the self-assembly process is based on the relative concentration between the terminating elements on the terminating components and the number of binding regions on the binding components. When the concentration of terminating components reaches a sufficient level, the self-assembly of the three components results in formation of a discrete particle, rather than a crosslinked network or hydrogel. A benefit of this supramolecular approach to producing nanoparticles is that the size of the final particles may be readily adjusted by adjusting the relative concentrations of the components in the preparation mixture.

In some embodiments, the terminating elements are the same as the binding elements. In some embodiments, the terminating component has a single terminating element that binds to one of the binding regions on the binding component. In these cases, each terminating component has only one terminating element. The terminating element is a chemical moiety that binds to the binding region of the binding component by one or more intermolecular forces. These terminating components bind to only one binding region on the binding component. In this way, crosslinking between the terminating component and the binding component may be avoided.

In some embodiments, the terminating component is a polymer, polypeptide, oligosaccharide or small molecule, so long as the terminating component binds to a binding region of the binding component. In some embodiments, the terminating component is a polymer that is derivatized with a terminating element. In some embodiments, the terminating elements are the same as the binding elements. For example, in some embodiments, the binding elements are adamantane and the terminating elements are adamantane. In some embodiments, the terminating component is poly(ethyleneglycol) derivatized with a binding element, such as adamantane.

In some embodiments, the SMNP may have two or more terminating components. In these embodiments, the supramolecular structure may have 2, 3, 4, 5, or 6 different terminating components. Each terminating component may have the same terminating element, or they may have different binding elements, but each terminating element will bind to a binding region of the binding component.

### Binding Component

The binding component has a plurality of binding regions that bind to the structural component, the magnetic nanoparticle, and the terminating component (e.g., to binding elements of the structural component, binding elements of the magnetic nanoparticle, and binding elements of the terminating component, respectively). The binding region is a chemical moiety that binds to the binding element by one or more intermolecular forces.

In some embodiments, two or more different binding components may be used, so long as both have binding regions that bind to the structural component, the magnetic nanoparticle, and the terminating component.

In some embodiments, the binding component is a polymer, oligosaccharide, or polypeptide. Any suitable material may be used that includes a plurality of binding regions. In some embodiments, the binding component is a polymer. In some embodiments, the binding component is polyethylene imine or branched polyethylene imine derivatized with a plurality of binding regions. A specific example of a binding component is a branched polyethylene imine derivatized with β-cyclodextrin. Another example of a binding component is poly-L-lysine derivatized with β-cyclodextrin.

### Molecular Recognition

In some embodiments, the binding regions and/or binding elements and/or terminating elements are molecular recognition elements. In other words, a binding region forms a molecular recognition pair with a binding element on the structural component or the magnetic nanoparticle, or with a terminating element on the terminating component.

Molecular recognition refers to the specific interaction between two or more molecules through one or more intermolecular forces. The molecules involved in molecular recognition exhibit molecular complementarity, and are called a molecular recognition pair or host-guest complex. In this case, the terms "host" and "guest" do not impart any particular relationship, but only describe two compounds which exhibit molecular complementarity, i.e.; bind to each other by molecular recognition. A "host" and a "guest" bind to each other, while two "host" compounds do not. Molecular recognition is a specific interaction, meaning that each molecular recognition element will bind to complementary molecules having particular structural features. In general, molecular recognition pairs bind more tightly than non-specific binding, since multiple interactions occur between the two molecular recognition elements.

Examples of molecular recognition pairs include small molecule host-guest complexes (including but not limited inclusion complexes), pairs of complementary oligonucleotide sequences (e.g. DNA-DNA, DNA-RNA or RNA-RNA that bind to each other by hybridization), antibody-antigen, protein-substrate, protein-inhibitor, and protein-protein interactions (such as α-helical peptide chains and β-sheet peptide chains).

In some embodiments, the SMNP self-assembles by molecular recognition. In this case, binding regions on the binding component form a molecular recognition pair with binding elements on the structural component and with binding elements on the magnetic nanoparticle. Terminating elements on the terminating component also bind to binding regions on the binding component to form a molecular recognition pair. The molecular recognition pairs formed between the binding component and the structural component, between the binding component and magnetic nanoparticle, and between the binding component and the terminating component, may be the same, or they may be different. In other words, the binding element on the structural component, the binding element on the magnetic nanoparticle, and the terminating element on the terminating component, each may be the same, or they may be different, but each binding elements binds to the same binding region on the binding component.

Specific examples of molecular recognition pairs include adamantane-α-cyclodextrin complexes or diazobenzene-β-cyclodextrin complexes. Other molecular recognition pairs include molecular complexes (e.g., steroid, pyrene, rhodamine, or doxorubicin in cyclodextrin). Other examples of molecular recognition pairs include biotin-streptavidin and complementary oligonucleotides.

### Functional Elements

In some embodiments, the SMNP further includes a functional element. In some embodiments, at least one of the structural component, magnetic nanoparticle, binding component, or terminating component further includes a functional element. In other embodiments, the functional element is a distinct element incorporated in the SMNP, e.g. via encapsulation and/or intermolecular forces. A functional element is a chemical moiety that imparts an additional function or activity to the SMNP that is not present when the functional element is missing. In some embodiments, the functional element is a light emitting (i.e. fluorescent or phosphorescent) compound. Fluorescent and phosphorescent labeled supramolecular structures may be used, for example in imaging studies in vitro or in vivo. In other embodiments, the functional element may be a compound having a radioactive or magnetically active isotope. For example, positron emitting isotopes, such as ⁶⁴Cu may be used to measure biodistribution of the supramolecular structures. Other suitable isotopes will be readily apparent to one of ordinary skill.

In some embodiments, the functional element is a targeting element that functions to target the supramolecular structure to particular cells. Such targeting elements include peptides, oligonucleotides, antibodies, and small molecules that bind to cell surface proteins. In general, any chemical moiety that specifically binds to one or more cell surface protein may be incorporated into the supramolecular structure. The cell surface proteins may be, for example, proteins on cancer cells or on bacteria or fungi. Specific examples of cell targeting moieties include RGD and EGF, folic acid, transferrin, and antibodies for targeting cell surface markers (e.g., Herceptin for Her2 on breast cancer cells).

In some embodiments, the functional element is a cell permeation element, that functions to increase cell membrane permeation. Specific examples of ligands that increase cell membrane permeation include the TAT ligand. Other cell membrane permeation ligands may also be used.

In some embodiments, the SMNP has two or more functional elements. For example, the supramolecular structure may have two targeting elements, increasing cell targeting selectivity, or increasing binding affinity by targeting more than one cell surface protein. Other examples include supramolecular structures having a targeting element and a cell permeation element, combining the effects of improved cell targeting and increased cell permeation. Yet another example may be a SMNP having an imaging element (light emitting or radioisotope) and targeting element for imaging targeted cells. Other combinations, such as two targeting elements and a cell permeation element, two targeting elements and a visualizing element, etc. may be readily envisioned.

In some embodiments, the SMNP includes two or more terminating components, each of which may further include a functional element. In this way, multiple functional elements may be incorporated by using multiple terminating components. For example, an SMNP may have a terminating component having no functional element and a terminating component having a targeting element. Terminating components having no functional element may be exchanged with terminating components having a functional element by treating the SMNP with a second terminating component or mixture of other terminating components. Likewise, the supramolecular structure may be prepared using a mixture of terminating components, each of which will be incorporated into the SMNP.

### Cargo

The supramolecular structure of the invention further includes a cargo that is an anti-cancer drug. The cargo is a chemical moiety encapsulated within the supramolecular structure and released from the supramolecular structure. Cargo materials may bind to one or more of the structural component, magnetic nanoparticle, binding component or terminating component, but do not interfere with self-assembly of the nanoparticle because they do not bind specifically to the binding regions of the binding component. The cargo compound may be a small molecule, such as a therapeutic compound (such as doxorubicin, taxol, rapamycin, cis-platin, or other anti-cancer agent for cancer therapy), protein, peptide, oligonucleotide (such as siRNA), or plasmid (for gene delivery). The supramolecular structures may deliver therapeutic proteins and oligonucleotides to a target cell, protecting the therapeutic compounds, proteins or oligonucleotides from degradation prior to delivery.

In some embodiments, the supramolecular structure may include two or more cargos. In some instances, two or more therapeutic compounds may be incorporated, allowing for delivery of a defined ratio of therapeutic compounds to a cell by adjusting the ratio of the therapeutic compound in the supramolecular structure. In other instances, a plasmid and small molecule may be incorporated. Other combinations may also be used.

### Preparation

The present dislcosure includes methods for preparing the supramolecular structures described above by preparing a suspension of structural components, magnetic nanoparticles, and binding components; and adding terminating components to said suspension. The ratio of an amount of structural components to magnetic nanoparticles to binding components to terminating components are selected in accordance with a predetermined size of said supramolecular structures. The structural components, magnetic nanoparticles, binding components, and terminating components self-assemble into said SMNPs having substantially said predetermined size. In some embodiments, the predetermined size is at least about 30 nm and less than about 500 nm.

The supramolecular structures may be readily prepared by combining the components together. The components self-assemble into the SMNP. Additional components (structural, binding or terminating) or cargo compounds may also be used, so long as the minimum elements are present. The additional components may include one or more functional elements and/or cargos.

After the supramolecular structure is formed, components may be exchanged with other components bearing appropriate binding elements, terminating elements or binding regions by treating the SMNP with additional components. For example, terminating components may be exchanged by treating the supramolecular structure with other terminating components (for example, bearing a functional element). Likewise, structural components, magnetic nanoparticles, or binding components may be exchanged by treating the SMNP with additional structural components, magnetic nanoparticles, or binding components. A suspension or solution of the components may be sonicated to accelerate or assist in component exchange reactions.

The size of the SMNPs may be easily adjusted by varying the ratios between the components used to prepare the supramolecular structures. A wide variety of SMNPs of different sizes may be easily prepared. This also enables combinatorial synthesis, as arrays of SMNPs may be assayed based on their specific function to optimize their activity.

Using component exchange, the size of the SMNPs may be adjusted after the supramolecular structures are formed by treating the pre-formed SMNPs with additional components. For example, if the pre-formed SMNPs is treated with additional binding component, the size will decrease. If the pre-formed supramolecular structure is treated with additional structural component, the size will increase.

The supramolecular structures can be disassociated *in vitro* and *in vivo* environments according to some embodiments of the current invention.

Functional elements may also be easily adjusted using this method. In many cases, components bearing a functional element may be included in the mixture used to prepare the SMNP. The extent to which the functional elements are present in the SMNP may be readily adjusted by changing the ratio between components having a functional element and components without. For example, if the functional element is present on the binding component, the ratio of the binding component having the functional element and the binding component lacking the functional element determines the extent to which the functional element is present in the SMNP formed. The same holds true when the functional element is present on the terminating component or structural component or magnetic nanoparticle.

When functional elements are present on terminating components, previously assembled SMNPs may be treated with terminating component(s) having a functional element. A portion of the terminating components will exchange to produce an SMNP bearing the functional element(s). Multiple terminating components bearing multiple different functional elements may be added in a similar manner. The extent to which the functional element is present on the resulting SMNP is determined by the concentration of the terminating component used to treat the pre-formed SMNP.

Individual components may be readily prepared using chemistry known in the art. The binding elements, terminating elements, and binding regions are selected based on the type of intermolecular forces desired for binding the components together, and may be selected at will. Molecular recognition provides numerous examples of chemical moieties that may be used as binding elements, terminating elements, or binding regions. For structural components, inorganic cores may be derivatized using methods known in the art to provide binding elements on the surface, when needed. Organic compounds, such as polymers and dendrimers, may be synthesized with suitable binding elements. Alternatively, organic cores, including polymers, dendrimers, polypeptides, etc. may be prepared bearing reactive functional groups, that may be derivatized with suitable binding elements or binding regions as desired.

Numerous methods exist for derivatizing organic compounds with suitable binding elements. For example, reactive functional groups on organic compounds, such as hydroxyls, thiols, amines, carboxylic acids, halides, alkenes, alkynes, azides, and others may be reacted or activated to react with a variety of other functional groups to form covalent bonds. For example, amine-bearing compounds having a free NH₂ group may be reacted with binding elements bearing amine-reactive groups such as isocyanates, isothiocyanates, and activated esters, such as N-hydroxysuccinimide (NHS) esters. In this way, binding elements may be readily added to any component. The number of binding elements on a particular component may be varied based on the number of reactive sites, and the amount of the reactive binding element used to prepare the component.

For example, amines on branched polyethyleneimine may be reacted with an activated cyclodextrin, such as tosylated cyclodextrin to prepare a binding component of polyethylene imine derivatized with cyclodextrin. Analogously, other polymers, such as poly-L-lysine, may be reacted with an activated cyclodextrin, such as tosylated cyclodextrin to prepare a component of poly-L-lysine derivatized with cyclodextrin binding elements. In other embodiments, amines, such as those in polyamidoamine dendrimers or in poly-L-lysine may be reacted with other activated binding elements, such as adamantine isocyanate, to prepare components derivatized with adamantane binding elements.

Chemistry commonly used to derivatize proteins may also be used to add binding elements to proteins, peptides, or antibodies. For example, amine-coupling or thiol-ene coupling can be used to generate irreversible bonds.

In some cases a linker may be required. Various bifunctional crosslinkers are known to those in the art for covalently bonding to proteins, any of which may be used. For example, heterodifunctional crosslinkers such as succinimidyl-4-[N-maleimidomethyl]cyclohexane-1-carboxylate (SMCC) and melaimidobutyryloxysuccinimide ester (GMBS) may be used to react with amines (via the succinimide esters), and then form a covalent bond with a free thiol (via the maleimide). Other crosslinkers, such as succinimidyl 3-(2-pyridyldithio)-propionate (SPDP) may react with amines (via the succinimide ester), and form a covalent bond with a free thiol via thiol exchange. Other difunctional crosslinkers include suberic acid bis(N-hydrosuccinimide ester), which can react with two amines. Other bifunctional and heterobifunctional crosslinkers useable with various surface modifications will be evident to those of skill in the art.

In some embodiments of the invention, it is desirable to include a reversible (cleavable) linker, a variety of which will be evident to a skilled worker. For example, 4-allyloxy-4-oxo-butanoic acid has an alkene group on one end that can be used for thiol-ene coupling to thiol, and its other end is a carboxylic group that can be coupled to an amine. There is an ester group in the middle of the linker that should hydrolyze slowly over time under physiological conditions. Other cleavable cross-linkers will be evident to a skilled worker. These include, e.g., disulfide bonds which will cleave upon reduction.

### Uses

The SMNPs have a variety of uses, particularly in biological applications. The simple methods required to produce the SMNPs enable rapid preparation of SMNPs of various sizes, or bearing specific functional elements. The use of different materials for structural, binding, and terminating components, and magnetic nanoparticles, enables a wide variety of utilities.

In some embodiments, the cargo can be released from the SMNP in a controllable manner. The time, location, and/or degree to which the cargo is released may be controlled. Without intending to be bound by theory, the cargo can be released by dissociating the SMNPs. The SMNPs can be disassociated *in vitro* and *in vivo* environments according to some embodiments. In some embodiments, the SMNPs are exposed to an external time-varying magnetic field, also called an alternating magnetic field (AMF). Applying the AMF causes an increase the local temperature in the vicinity of the magnetic nanoparticles, dissociating the SMNP and releasing the cargo. The local temperature in the vicinity of the magnetic nanoparticles can be increased without raising the temperature of surrounding solution. In this way, the cargo can be delivered and released without detrimental effects of increased temperature in the surrounding effects, for example, when the cargo is delivered to cells (whether in vitro or in vivo).

The disclosure includes methods of delivering therapeutic compounds by treating a cell with an SMNP described herein, having a therapeutic compound as cargo. The therapeutic compound may be, for example, a protein or peptide (including antibodies), an oligonucleotide (e.g., siRNA) or a small molecule. The small molecule may be, for example, an anti-cancer agent (e.g. doxorubicin, taxol, paclitaxel, cis-platin, rapamycin, or other anti-cancer agent), antibiotic, anti-bacterial, or anti-fungal agent. Functional elements on the supramolecular structure may improve cell targeting, internalization, or distribution. More than one therapeutic compound may be delivered in a single SMNP, and if desired, the ratio of therapeutic compounds may be controlled.

Also disclosed are methods of using the SMNP described herein for magnetic sorting of cells, proteins, peptides or other materials. Targeting functional elements allow the SMNP to bind to specific cells, proteins, peptides, or other materials, facilitating magnetic separation.

The SMNPs may be used for gene therapy (*in vivo*) or for cellular transfection (*in vitro*) by delivering genes or plasmids to cells.

Embodiments include methods of delivering a gene to a cell by contacting the cell with a SMNP described herein, bearing a plasmid cargo. Treating the cell with the supramolecular structure results in internalization of the SMNP, followed by release of the plasmid into the cell. This can result in effective "transfection" of the targeted cell with the plasmid of interest. In general, any plasmid, bearing any gene may be introduced into the cell in this manner. Likewise, targeting and/or cell permeation elements may improve cell specificity and/or internalization.

Other methods of using the SMNP described herein include methods of photothermotherapy by treating cells with supramolecular structures described herein having gold nanoparticles as structural components.

The supramolecular nanoparticles may be used for molecular imaging (e.g. PET), using components bearing functional elements having one or more suitable isotopes or light emitting compounds. Likewise, supramolecular structures may be used for radiotherapy where one or more components includes a functional element having a therapeutic isotope. Cell targeting and cell permeation functional elements may further improve the effectiveness of these supramolecular structures.

### Pharmaceutical Compositions

The SMNPs discussed herein can be formulated into various compositions, for use in diagnostic or therapeutic treatment methods. The compositions (*e.g*. pharmaceutical compositions) can be assembled as a kit. Generally, a pharmaceutical composition of the invention comprises an effective amount (*e.g*., a pharmaceutically effective amount) of a composition of the invention.
of the SMNP structure for use according to the invention can be formulated as a pharmaceutical composition, which further comprises a pharmaceutically acceptable carrier. By a "pharmaceutically acceptable carrier" is meant a material that is not biologically or otherwise undesirable, *i.e.,* the material may be administered to a subject without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art. For a discussion of pharmaceutically acceptable carriers and other components of pharmaceutical compositions, see, *e.g.*, Remington's Pharmaceutical Sciences, 18th ed., Mack Publishing Company, 1990. Some suitable pharmaceutical carriers will be evident to a skilled worker and include, *e.g.*, water (including sterile and/or deionized water), suitable buffers (such as PBS), physiological saline, cell culture medium (such as DMEM), artificial cerebral spinal fluid, or the like.

A pharmaceutical composition or kit can contain in addition other pharmaceuticals. The other agent(s) can be administered at any suitable time during the treatment of the patient, either concurrently or sequentially.

One skilled in the art will appreciate that the particular formulation will depend, in part, upon the particular agent that is employed, and the chosen route of administration. Accordingly, there is a wide variety of suitable formulations of compositions for use according to the present invention.

Formulations which are suitable for topical administration directly in the CNS include, *e.g*., suitable liquid carriers, or creams, emulsions, suspensions, solutions, gels, creams, pastes, foams, lubricants, or sprays. Topical administration in the CNS is possible when the CNS is opened by wound or during a surgery.

One skilled in the art will appreciate that a suitable or appropriate formulation can be selected, adapted or developed based upon the particular application at hand. Dosages for compositions of the invention can be in unit dosage form. The term "unit dosage form" as used herein refers to physically discrete units suitable as unitary dosages for animal (*e.g*. human) subjects, each unit containing a predetermined quantity of an agent of the invention, alone or in combination with other therapeutic agents, calculated in an amount sufficient to produce the desired effect in association with a pharmaceutically acceptable diluent, carrier, or vehicle.

One skilled in the art can easily determine the appropriate dose, schedule, and method of administration for the exact formulation of the composition being used, in order to achieve the desired effective amount or effective concentration of the agent in the individual patient.

The dose of a composition, administered to an animal, particularly a human, in the context of the present invention should be sufficient to effect at least a detectable amount of a diagnostic or therapeutic response in the individual over a reasonable time frame. The dose used to achieve a desired effect will be determined by a variety of factors, including the potency of the particular agent being administered, the pharmacodynamics associated with the agent in the host, the severity of the disease state of infected individuals, other medications being administered to the subject, etc. The size of the dose also will be determined by the existence of any adverse side effects that may accompany the particular agent, or composition thereof, employed. It is generally desirable, whenever possible, to keep adverse side effects to a minimum. The dose of the biologically active material will vary; suitable amounts for each particular agent will be evident to a skilled worker.

Also disclosed is a kit useful for any of the methods disclosed herein, either *in vitro* or *in vivo.* Such a kit can comprise one or more of the compositions of the invention. Optionally, the kits comprise instructions for performing the method. Optional elements of a kit include suitable buffers, pharmaceutically acceptable carriers, or the like, containers, or packaging materials. The reagents of the kit may be in containers in which the reagents are stable, *e.g.,* in lyophilized form or stabilized liquids. The reagents may also be in single use form, *e.g.,* in single dosage form.

Previously, we demonstrated a convenient, flexible, and modular self-assembled synthetic approach for the preparation of supramolecular nanoparticle **(SNP)** vectors from a collection of molecular building blocks via a multivalent molecular recognition based on adamantane **(Ad)** and β-cyclodextrin **(CD)** motifs.⁽⁷⁾ Such a self-assembled synthetic strategy enables control over the sizes, surface chemistry and payloads of **SNP** vectors for many diagnostic and therapeutic applications such as positron emission tomography (PET) imaging,⁽⁷⁾ magnetic resonance imaging (MRI),⁽⁸⁾ photothermal treatment,⁽⁹⁾ as well as highly efficient delivery of genes,⁽¹⁰⁾ intact transcription factors,⁽¹¹⁾ and drug-polymer conjugates.⁽¹²⁾

Herein, magnetothermally responsive doxorubicin-encapsulated supramolecular magnetic nanoparticles **(DoxcSMNPs)** as a unique on-demand drug delivery/release system **(****FIG. 1****)** are described. The supramolecular synthetic strategy⁽⁷⁻¹²⁾ was employed to prepare size-controllable **DoxcSMNPs** from the fluorescent anti-cancer drug, **Dox,** as well as other four molecular building blocks, including **Ad**-grafted polyamidoamine dendrimers **(Ad-PAMAM),** β-**CD**-grafted branched polyethylenimine **(CD-PEI), Ad**-functionalized polyethylene glycol **(Ad-PEG)** and 6-nm **Ad**-grafted Zn_{0.4}Fe_{2.6}O₄ superparamagnetic nanoparticle **(Ad-MNP).** Tumor EPR effects are expected to drive preferential accumulation of **DoxcSMNPs** in tumor,^{(4, 13)} which constitutes the spatio-control within **DoxcSMNPs.** After **DoxcSMNPs** achieve maximum accumulation in tumor, an external time-varying magnetic field, also called an alternating magnetic field (AMF), is applied to disassemble **DoxcSMNPs,** leading to a burst release of **Dox.** According to our molecular design, the embedded magnetic NP **(Ad-MNP)** serves as a built-in heat transformer that coverts radiofrequency AMF into heat, representing a stimuli-responsive drug release mechanism within **DoxcSMNPs.** In order to determine the ideal size of **DoxcSMNPs** for the proper spatial distribution and optimal time point for the maximized tumor accumulation of **DoxcSMNPs, ⁶⁴Cu-labeled DoxcSMNPs** are prepared by incorporating radioisotope (i.e., ⁶⁴Cu) in the presence of DOTA ligand and then subjected to PET-based *in vivo* imaging studies. In parallel, an optimal AMF condition was determined by monitoring **Dox** release from **DoxcSMNPs** *in vitro.* Based on the results from both *in vivo* biodistribution and *in vitro* AMF optimization studies, we were able to design an *in vivo* treatment protocol for **DoxcSMNPs** to accomplish effective cancer therapy. Taken together, an acute level of drug concentration can be delivered to tumor with spatio-temporal control thus significantly reducing drug dosage.

### EXAMPLES

Recently, we have developed uniquely designed **MNPs** by adopting nonmagnetic dopants and core-shell structures, which exhibit superior magnetic hyperthermia properties over conventional **MNPs**.⁽¹⁴⁾ By utilizing one of these **MNPs,** a magnetically activated drug release system was demonstrated *in vitro,* in which a zinc-doped **MNP** was incorporated into mesoporous silica where drug molecules were released upon exposure to a magnetic field stimulus.⁽¹⁵⁾ The strong heat induction from zinc-doped **MNP,** due to its higher saturation magnetization value, makes this inorganic NP an ideal component to incorporate into our thermally responsive **SNP** vector.⁽¹⁶⁾ We modified the 6-nm zinc-doped **MNP** with **Ad** to make **Ad-MNP (****FIG. 2a****)** as one of molecular building blocks that can be self-assembled into **DoxcSMNPs.** By fine-tuning the different ratios of the molecular building blocks, three sizes of **DoxcSMNPs** are prepared (70, 100, and 160 nm, **FIG. 2b-****2d).** All three sizes of **DoxcSMNPs** have a narrow size distribution measured by light scattering, and **Dox** encapsulation efficiency is determined to be *ca.* 95% (see **Figure S2** in supporting information).

A key physical parameter that determines the overall biodistribution pattern and therapeutic performance is the sizes of **Dox⊂SMNPs**.^{(2b, 17)} We used micro-PET imaging to identify an optimal size of **DoxcSMNPs** with the highest retention in tumor. 70-nm, 100-nm, and 160-nm **⁶⁴Cu-labeled** (300 µCi) **DoxcSMNPs** were synthesized (see supporting information) and injected into DLD-1 tumor-bearing NU/NU mice via intravenous (*i.v*.) administration. The time dependent accumulation of the three **DoxcSMNPs** in the tumor versus whole body are summarized and plotted in **FIG. 2e****.** The results show that 70-nm **DoxcSMNPs** achieve their maximum tumor accumulation at 36 h post-injection, which is the critical time point for the optimal spatial distribution of **DoxcSMNPs.** The representative two-dimensional cross-sections of static filtered back-projection micro-PET images of the three **DoxcSMNPs** taken at 36 h post-injection are shown in **FIG. 2f-2h****,** indicating that 70-nm **DoxcSMNPs** have the highest tumor-specific uptake among the three sizes. We note that the high signal measured in the liver should not be a major concern since it is presumably due to demetalation of ⁶⁴Cu from the DOTA ligand,⁽¹⁸⁾ and thus does not accurately represent the location of **DoxcSMNPs** in that organ. An *ex vivo* biodistribution study using a gamma radiation counter at the termination of the experiment (48 h post-injection, **FIG. 2i****,** details in supporting information) also confirm the same conclusion as PET-imaging results; therefore, 70-nm **DoxcSMNPs** were utilized for further *in vitro* and *in vivo* studies.

The self-assembly of **Ad-PAMAM, Ad-MNP, CD-PEI,** and **Ad-PEG** generates **SMNP** vectors with intraparticular cationic hydrogel networks. Such hydrogel networks constitute a unique nano-environment that induces self-organization of **Dox** molecules driven by their intermolecular π-π stacking interactions.⁽¹⁹⁾ As a result, the fluorescent signal of encapsulated **Dox** molecules is quenched remarkably (*ca*. 97%, see **Figure S3** in supporting information) while associated with the **SMNP** vector. AMF is used as an external on-demand control that triggers the burst release of encapsulated **Dox** from the disassembled **SMNP** vector via magnetic heating.⁽⁹⁾ Once released from the **SMNP** vector, the fluorescence of **Dox** molecules is restored. We use this photophysical property of **Dox** to investigate **DoxcSMNPs'** magnetically activated drug release performance as a function of AMF duration (500 kHz, 37.4 kA/m). Results show that the drug release from **DoxcSMNPs** nearly saturates with 10 min of AMF without raising the temperature of surrounding solution (details in supporting information **Figure S4**). When we apply multiple AMF pulses for a 2-min duration to the **DoxcSMNPs** with an 8-min interval **(****FIG. 3a****),** approximately 50% of encapsulated **Dox** molecules are released in the first AMF pulse (**FIG**. **3a****,** red line), while more **Dox** is released in a stepwise fashion after subsequent AMF pulses up to 7 or 8 pulses (50 min; **FIG. 3a****,** black line). Based on these results, we selected a single pulse of AMF application with a 10-min duration as an effective AMF condition of **DoxcSMNPs**-based drug delivery system in further *in vitro* and *in vivo* studies, which can achieve on-demand release of an acute level of **Dox** concentration while avoiding unregulated drug release and thermal heating of surrounding medium.

*In vitro* on-demand release of **Dox** from 70-nm **DoxcSMNPs** were investigated in DLD-1 colorectal adenocarcinoma cell line with (**FIG**. **3b****,** left column) and without (**FIG. 3b****,** right column) the application of a 10-min AMF (500 kHz, 37.4 kA/m). After the cells (1.5 x 10⁴) are transfected with 70-nm **DoxcSMNPs** (200 µg/ml treatment), minimal drug release (Dim **Dox** fluorescence) and cell damage are observed (**FIG**. **3b****,** right column). However, after exposure to AMF, blebbing and **Dox** fluorescence (red) is increased (**FIG**. **3b****,** left column). Also, nucleus fragmentations⁽²⁰⁾ and formation of apoptotic cell bodies are seen, demonstrating the consequence of effective **Dox** release from **DoxcSMNPs** under AMF application. A CCK-8 assay is used to quantify cell viability showing the decrease of viability to 30% after AMF application. Without the application of AMF, negligible cytotoxicity is observed and AMF alone has no effect on cell viability **(****FIG. 3c****).**

Based on the systemic biodistribution results (optimal time point, i.e., 36 h post-injection, **FIG. 2e**) and the *in vitro* drug release experiments (favorable AMF condition, i.e., 10 min, **FIG. 3**), we designed an idealized *in vivo* treatment protocol of 70-nm **DoxcSMNPs** for cancer therapy. When the tumor volume of DLD-1 xenografted mice (n=3) reached 100 mm³, **DoxcSMNPs** (70 nm, 150 µg/kg) were administered intravenously (day 0) followed by AMF treatment (10 min, 500 kHz, 37.3 kA/m) after 36 h post-injection. Anti-tumor efficacy results treated with **DoxcSMNPs** (w/ and w/o AMF) and other control studies (i.e., AMF only and PBS only) are summarized as plots of tumor volume over the course of treatment in **FIG. 4a****.** The control groups (i.e., **DoxcSMNPs** w/o AMF, AMF only, and PBS) do not show any statistically significant differences in tumor suppression **(****FIG. 4a****).** The group treated with a single injection of **DoxcSMNPs** with applied AMF shows tumor suppression efficacy only up to day 7 (**FIG**. **4a****,** red line). In contrast, the group treated with a double injection (day 0 and day 7) of **DoxcSMNPs** with AMF shows continued and effective inhibition of tumor growth (**FIG**. **4a****,** black line). The tumor images of each group are shown in **FIG. 4b****,** which visually confirm the effective tumor suppression of the doubly injected **DoxcSMNPs** with AMF application. In addition, the drug-free vector (**SMNPs** w/o **Dox)** was administered following the same protocol, which gives similar results as control groups indicating that the effect of thermal heating from **SMNP** is negligible (see supporting information). It is notable that our **DoxcSMNPs** system only requires a minute amount of drug (2.8 µg/kg **Dox** per injection) for tumor suppression, which is 3 orders of magnitude lower than other NP systems (see supporting information, **Table 1**).⁽²¹⁾

An on-demand drug delivery/release system that utilizes magnetothermally responsive **DoxcSMNPs** for highly effective *in vivo* cancer treatment has been demonstrated. An optimized *in vivo* treatment protocol of **DoxcSMNPs** was designed after studying their biodistribution at a systemic level and evaluating *in vitro* trigger release of **Dox** with an optimal AMF condition.

The examples disclosed below are provided to illustrate the invention but not to limit its scope. Other variants of the invention will be readily apparent to one of ordinary skill in the art and shall be considered as falling within the scope of the invention insofar as they fall within the scope of the appended claims.

### REFERENCES

(1)T. M. Allen, P. R. Cullis, Science 2004, 303, 1818-1822.
(2) a) M. E. Davis, Z. Chen, D. M. Shin, Nat. Rev. Drug. Discov. 2008, 7, 771-782; b) R. A. Petros, J. M. DeSimone, Nat. Rev. Drug. Discov. 2010, 9, 615-627; c) A. Z. Wang, R. Langer, O. C. Farokhzad, Ann. Rev. Med, 2012, 63, 185-198; d) C. Tassa, S. Y. Shaw, R. Weissleder, Acc. Chem. Res. 2011, 44, 842-852.
(3) V. Torchilin, Adv. Drug. Deliver. Rev. 2011, 63, 131-135.
(4) a) Y. Matsumura, H. Maeda, Cancer Res. 1986, 46, 6387-6392; b) S. Dufort, L. Sancey, J. L. Coll, Adv. Drug. Deliver. Rev. 2012, 64, 179-189.
(5) a) J. Ge, E. Neofytou, T. J. Cahill, R. E. Beygui, R. N. Zare, ACS Nano 2012, 6, 227-233; b) H. Yan, C. Teh, S. Sreejith, L. Zhu, A. Kwok, W. Fang, X. Ma, K. T. Nguyen, V. Korzh, Y. Zhao, Angew. Chem. Int. Ed. 2012, 51, 8373-8377.
(6) a) T. J. Harris, G. von Maltzahn, A. M. Derfus, E. Ruoslahti, S. N. Bhatia, Angew. Chem. Int. Ed. 2006, 45, 3161-3165; b) G. Wu, A. Mikhailovsky, H. A. Khant, C. Fu, W. Chiu, J. A. Zasadzinski, J. Am. Chem. Soc. 2008, 130, 8175-8177; c) A. Desert, I. Chaduc, S. Fouilloux, J. C. Taveau, O. Lambert, M. Lansalot, E. Bourgeat-Lami, A. Thill, O. Spalla, S. Ravaine, E. Duguet, Poly. Chem. 2012, 3, 1130-1132; d) A. Agarwal, M. A. Mackey, M. A. El-Sayed, R. V. Bellamkonda, ACS Nano 2011, 5, 4919-4926.
(7) H. Wang, S. T. Wang, H. Su, K. J. Chen, A. L. Armijo, W. Y. Lin, Y. J. Wang, J. Sun, K. Kamei, J. Czernin, C. G. Radu, H. R. Tseng, Angew, Chem. Int. Ed. 2009, 48, 4344-4348.
(8) K. J. Chen, S. M. Wolahan, H. Wang, C. H. Hsu, H. W. Chang, A. Durazo, L. P. Hwang, M. A. Garcia, Z. K. Jiang, L. Wu, Y. Y. Lin, H. R. Tseng, Biomaterials 2011, 32, 2160-2165.
(9) S. T. Wang, K. J. Chen, T. H. Wu, H. Wang, W. Y. Lin, M. Ohashi, P. Y. Chiou, H. R. Tseng, *Angew. Chem. Int. Ed.* **2010,** *49*, 3777-3781.
(10) a) H. Wang, K. J. Chen, S. T. Wang, M. Ohashi, K. I. Kamei, J. Sun, J. H. Ha, K. Liu, H. R. Tseng, *Chem. Commun.* **2010,** *46*, 1851-1853; b) H. Wang, K. Liu, K. J. Chen, Y. J. Lu, S. T. Wang, W. Y. Lin, F. Guo, K. I. Kamei, Y. C. Chen, M. Ohashi, M. W. Wang, M. A. Garcia, X. Z. Zhao, C. K. F. Shen, H. R. Tseng, *ACS Nano* **2010,** *4*, 6235-6243; c) K. Liu, H. Wang, K. J. Chen, F. Guo, W. Y. Lin, Y. C. Chen, D. L. Phung, H. R. Tseng, C. K. F. Shen, *Nanotechnology* **2010,** *21*.
(11) Y. Liu, H. Wang, K. Kamei, M. Yan, K. J. Chen, Q. H. Yuan, L. Q. Shi, Y. F. Lu, H. R. Tseng, *Angew. Chem. Int. Ed.* **2011,** *50*, 3058-3062.
(12) K. J. Chen, L. Tang, M. A. Garcia, H. Wang, H. Lu, W. Y. Lin, S. Hou, Q. Yin, C. K. F. Shen, J. J. Cheng, H. R. Tseng, Biomaterials 2012, 33, 1162-1169.
(13) S. Acharya, S. K. Sahoo, Adv. Drug. Deliver. Rev. 2011, 63, 170-183.
(14) a) D. Yoo, J. H. Lee, T. H. Shin, J. Cheon, Acc. Chem. Res. 2011, 44, 863-874; b) J. H. Lee, J. T. Jang, J. S. Choi, S. H. Moon, S. H. Noh, J. W. Kim, J. G. Kim, I. S. Kim, K. I. Park, J. Cheon, Nat. Nanotechnol. 2011, 6, 418-422; c) S. H. Noh, W. Na, J. T. Jang, J. H. Lee, E. J. Lee, S. H. Moon, Y. Lim, J. S. Shin, J. Cheon, Nano Lett. 2012, 12, 3716-3721.
(15) C. R. Thomas, D. P. Ferris, J. H. Lee, E. Choi, M. H. Cho, E. S. Kim, J. F. Stoddart, J. S. Shin, J. Cheon, J. I. Zink, J. Am. Chem. Soc. 2010, 132, 10623-10625.
(16) J. T. Jang, H. Nah, J. H. Lee, S. H. Moon, M. G. Kim, J. Cheon, Angew. Chem. Int. Ed. 2009, 48, 1234-1238.
(17) H. Cabral, Y. Matsumoto, K. Mizuno, Q. Chen, M. Murakami, M. Kimura, Y. Terada, M. R. Kano, K. Miyazono, M. Uesaka, N. Nishiyama, K. Kataoka, Nat. Nanotechnol. 2011, 6, 815-823.
(18) C. A. Boswell, X. K. Sun, W. J. Niu, G. R. Weisman, E. H. Wong, A. L. Rheingold, C. J. Anderson, J. Med. Chem. 2004, 47, 1465-1474.
(19) a) V. Y. Erukova, O. O. Krylova, Y. N. Antonenko, N. S. Melik-Nubarov, BBA-Biomembranes 2000, 1468, 73-86; b) E. Hayakawa, K. Furuya, H. Ueno, T. Kuroda, M. Moriyama, A. Kondo, Chem. Pharm. Bull. 1991, 39, 1009-1012; c) M. K. Yu, Y. Y. Jeong, J. Park, S. Park, J. W. Kim, J. J. Min, K. Kim, S. Jon, Angew. Chem. Int. Ed. 2008, 47, 5362-5365.
(20) W. Zoli, P. Ulivi, A. Tesei, F. Fabbri, M. Rosetti, R. Maltoni, D. C. Giunchi, L. Ricotti, G. Brigliadori, I. Vannini, D. Amadori, Breast Cancer Res. 2005, 7, R681-R689.
(21) a) W. Zhang, Z. Y. Guo, D. Q. Huang, Z. M. Liu, X. Guo, H. Q. Zhong, Biomaterials 2011, 32, 8555-8561; b) A. Agarwal, M. A. Mackey, M. A. El-Sayed, R. V. Bellamkonda, ACS Nano 2011, 5, 4919-4926; c) Z. Liu, A. C. Fan, K. Rakhra, S. Sherlock, A. Goodwin, X. Y. Chen, Q. W. Yang, D. W. Felsher, H. J. Dai, Angew. Chem. Int. Ed. 2009, 48, 7668-7672.
(22) WO/2010/099466
(23) U.S. Patent Application Publication No. 2013/0046274

## Claims

1. A supramolecular magnetic nanoparticle (SMNP) structure comprising an anti-cancer drug for use in a therapeutic method for treating cancer in a subject, the SMNP structure comprising:
a plurality of structural components each including a plurality of binding elements;
at least one magnetic nanoparticle each including a plurality of binding elements;
a plurality of binding components each including a plurality of binding regions, wherein each of the binding regions is adapted to bind to a binding element; and
a plurality of terminating components each including a terminating element, wherein the terminating element is adapted to occupy a binding region, wherein the plurality of terminating components are present in a sufficient quantity relative to the plurality of binding regions of the plurality of binding components to terminate further binding;
said anti-cancer drug being a cargo encapsulated within the SMNP structure; said therapeutic method comprising the delivery of said cargo to a subject, and further comprising:
imaging of the subject using positron electron tomography (PET) imaging;
measuring time dependent accumulation of the cargo within the supramolecular magnetic nanoparticles (SMNP) in tumor versus a whole body of the subject using PET imaging;
determining an optimal or critical point in time after administration of the SMNPs to the subject when the cargo within the SMNPs achieves maximum tumor accumulation; and
triggering a release of the encapsulated cargo from disassembled SMNPs by using an alternating magnetic field (AMF).

2. The supramolecular structure for use according to claim 1, wherein said plurality of structural components in said supramolecular structure comprises at least one of a dendrimer, branched polyethyleneimide, linear polyethyleneimide, polylysine, polylactide, polylactide-co- glycolide, polyanhydrides, poly-e-caprolactones, polymethyl methacrylate, poly(N-isopropyl acrylamide) or polypeptides.

3. The supramolecular structure for use according to claim 2, wherein said plurality of structural components comprises a dendrimer.

4. The supramolecular structure for use according to any one of claims 1, 2 or 3, wherein said plurality of terminating components comprises at least one of polyethylene glycol, an adamantane derivative, target ligands, peptides, antibodies or proteins.

5. The supramolecular structure for use according to any one of claims 1 and 2-4, wherein the supramolecular magnetic nanoparticle (SMNP) has a predetermined size of at least 30 nm and less than 500 nm.

6. The supramolecular structure for use according to any one of claims 1 and 2-5, wherein the binding regions bind to the terminating components or structural components to form a molecular recognition pair selected from the group consisting of antibody-antigen; protein-substrate; protein-inhibitor; protein-protein; a pair of complementary oligonucleotides; and an inclusion complex.

7. The supramolecular structure for use according to claim 6, wherein the molecular recognition pair is an inclusion complex, preferably adamantane-βcyclodextrin or diazobenzene-α-cyclodextrin.

8. The supramolecular structure for use according to any one of claims 1 and 2-7, wherein at least one of the structural component, binding component, magnetic nanoparticle, or terminating component further comprises a functional element.

9. The supramolecular structure for use according to claim 8, wherein the functional element is a targeting ligand or cell permeation ligand.

10. The supramolecular structure for use according to any one of claims 1 and 2-9, wherein a further cargo is siRNA; peptide; oligonucleotide; or plasmid.

11. The supramolecular structure for use according to any of claims 1 to 10, wherein the AMF is selected to increase a local temperature in the vicinity of the magnetic nanoparticles.

12. The supramolecular structure for use according to anyone of claims 1 to 11, wherein the cargo is substantially retained by the supramolecular structure of the SMNP prior to applying the AMF.

13. The supramolecular structure for use according to anyone of claims 1 to 12, wherein the SMNP further comprises a functional element selected from a targeting ligand and a cell permeation ligand.

14. The supramolecular structure for use according to any of claims 1 to 13 wherein said method further comprises administering the SMNPs to the subject prior to determining the distribution of the SMNPs within the body of the subject.

## Patentansprüche

1. Supramolekulare magnetische Nanoteilchen (SMNP)-Struktur, die einen Anti-krebs-Arzneistoff umfasst, zur Verwendung in einem therapeutischen Verfahren zur Behandlung von Krebs in einem Lebewesen, wobei die SMNP-Struktur umfasst:
eine Mehrzahl von strukturellen Komponenten, die jeweils eine Mehrzahl von Bindungselementen umfassen;
mindestens ein magnetisches Nanoteilchen, das oder die jeweils eine Mehrzahl von Bindungselementen umfasst oder umfassen;
eine Mehrzahl von Bindungskomponenten, die jeweils eine Mehrzahl von Bindungsbereichen umfassen, wobei jeder der Bindungsbereiche zum Binden an ein Bindungselement angepasst ist; und
eine Mehrzahl von Abbruchkomponenten, die jeweils ein Abbruchelement umfassen, wobei das Abbruchelement zum Besetzen eines Bindungsbereichs angepasst ist, wobei die Mehrzahl von Abbruchkomponenten in einer ausreichenden Menge relativ zu der Mehrzahl von Bindungsbereichen der Mehrzahl von Bindungskomponenten vorliegt, so dass ein weiteres Binden abgebrochen wird;
wobei der Antikrebs-Arzneistoff eine Beladung ist, die innerhalb der SMNP-Struktur eingekapselt ist; wobei das therapeutische Verfahren die Abgabe der Beladung an ein Lebewesen umfasst, und wobei es ferner umfasst:
Durchführen einer Bildgebung des Lebewesens unter Verwendung einer Positronen-Elektronen-Tomographie (PET)-Bildgebung;
Messen der zeitabhängigen Ansammlung der Beladung innerhalb der supramolekularen magnetischen Nanoteilchen (SMNP) in einem Tumor gegenüber dem gesamten Körper des Lebewesens unter Verwendung der PET-Bildgebung;
Bestimmen eines optimalen oder kritischen Zeitpunkts nach der Verabreichung der SMNPs an das Lebewesen, wenn die Beladung innerhalb der SMNPs eine maximale Tumoransammlung erreicht; und
Auslösen einer Freisetzung der eingekapselten Beladung von zerfallenen SMNPs unter Verwendung eines alternierenden Magnetfelds (AMF).

2. Supramolekulare Struktur zur Verwendung nach Anspruch 1, wobei die Mehrzahl von strukturellen Komponenten in der supramolekularen Struktur mindestens eines von einem Dendrimer, verzweigtem Polyethylenimid, linearem Polyethylenimid, Polylysin, Polylaktid, Polylaktid-co-glykolid, Polyanhydriden, Poly-ε-caprolaktonen, Polymethylmethacrylat, Poly(N-isopropylacrylamid) oder Polypeptiden umfasst.

3. Supramolekulare Struktur zur Verwendung nach Anspruch 2, wobei die Mehrzahl von strukturellen Komponenten ein Dendrimer umfasst.

4. Supramolekulare Struktur zur Verwendung nach einem der Ansprüche 1, 2 oder 3, wobei die Mehrzahl von Abbruchkomponenten mindestens eines von Poly-ethylenglykol, einem Adamantanderivat, Zielliganden, Peptiden, Antikörpern oder Proteinen umfasst.

5. Supramolekulare Struktur zur Verwendung nach einem der Ansprüche 1 und 2 bis 4, wobei das supramolekulare magnetische Nanoteilchen (SMNP) eine vorgegebene Größe von mindestens 30 nm und weniger als 500 nm aufweist.

6. Supramolekulare Struktur zur Verwendung nach einem der Ansprüche 1 und 2 bis 5, wobei die Bindungsbereiche an die Abbruchkomponenten oder strukturellen Komponenten zur Bildung eines molekularen Erkennungspaares binden, ausgewählt aus der Gruppe, bestehend aus Antikörper-Antigen; Protein-Substrat; Protein-Hemmstoff; Protein-Protein; einem Paar von komplementären Oligonukleotiden; und einem Einschlusskomplex.

7. Supramolekulare Struktur zur Verwendung nach Anspruch 6, wobei das molekulare Erkennungspaar ein Einschlusskomplex, vorzugsweise Adamantan-β-cyclodextrin oder Diazobenzol-α-cyclodextrin, ist.

8. Supramolekulare Struktur zur Verwendung nach einem der Ansprüche 1 und 2 bis 7, wobei mindestens eine(s) der strukturellen Komponente, der Bindungskomponente, des magnetischen Nanoteilchens oder der Abbruchkomponente ferner ein funktionelles Element umfasst.

9. Supramolekulare Struktur zur Verwendung nach Anspruch 8, wobei das funktionelle Element ein Zielsteuerungsligand oder ein Zellpermeationsligand ist.

10. Supramolekulare Struktur zur Verwendung nach einem der Ansprüche 1 und 2 bis 9, wobei eine weitere Beladung siRNA; Peptid; Oligonukleotid; oder Plasmid ist.

11. Supramolekulare Struktur zur Verwendung nach einem der Ansprüche 1 bis 10, wobei das AMF zum Erhöhen einer lokalen Temperatur in der Umgebung der magnetischen Nanoteilchen ausgewählt wird.

12. Supramolekulare Struktur zur Verwendung nach einem der Ansprüche 1 bis 11, wobei die Beladung durch die supramolekulare Struktur der SNMP vor dem Anwenden des AMF im Wesentlichen zurückgehalten wird.

13. Supramolekulare Struktur zur Verwendung nach einem der Ansprüche 1 bis 12, wobei das SMNP ferner ein funktionelles Element umfasst, das aus einem Zielsteuerungsliganden und einem Zellpermeationsliganden ausgewählt ist.

14. Supramolekulare Struktur zur Verwendung nach einem der Ansprüche 1 bis 13, wobei das Verfahren ferner das Verabreichen der SMNPs an das Lebewesen vor dem Bestimmen der Verteilung der SMNPs innerhalb des Körpers des Lebewesens umfasst.

## Revendications

1. Structure de nanoparticule magnétique supramoléculaire (SMNP) comprenant un médicament anticancéreux pour son utilisation dans un procédé thérapeutique pour traiter un cancer chez un sujet, la structure de SMNP comprenant :
une pluralité de composants structurels incluant chacun une pluralité d'éléments de liaison ;
au moins une nanoparticule magnétique incluant chacune une pluralité d'éléments de liaison ;
une pluralité de composants de liaison incluant chacun une pluralité de régions de liaison, dans laquelle chacune des régions de liaison est adaptée pour se lier à un élément de liaison ; et
une pluralité de composants de terminaison incluant chacun un élément de terminaison, dans laquelle l'élément de terminaison est adapté pour occuper une région de liaison, dans laquelle la pluralité de composants de terminaison sont présents en une quantité suffisante par rapport à la pluralité de régions de liaison de la pluralité de composants de liaison pour terminer la poursuite de la liaison ;
ledit médicament anticancéreux étant une cargaison encapsulée au sein de la structure de SMNP ; ledit procédé thérapeutique comprenant la délivrance de ladite cargaison à un sujet, et comprenant en outre :
l'imagerie du sujet à l'aide d'une imagerie par tomographie à émission de positons (TEP) ;
la mesure de l'accumulation de la cargaison en fonction du temps au sein des nanoparticules magnétiques supramoléculaires (SMNP) dans une tumeur par rapport à l'ensemble de l'organisme du sujet à l'aide de l'imagerie TEP ;
la détermination d'un moment donné optimal ou critique après l'administration des SMNPs au sujet auquel la cargaison au sein des SMNPs atteint une accumulation maximale dans la tumeur ; et
le déclenchement d'une libération de la cargaison encapsulée à partir des SMNPs désassemblées à l'aide d'un champ magnétique alternatif (AMF).

2. Structure supramoléculaire pour son utilisation selon la revendication 1, dans laquelle ladite pluralité de composants structurels dans ladite structure supramoléculaire comprend au moins l'un parmi un dendrimère, un polyéthylèneimide ramifié, un polyéthylèneimide linéaire, une polylysine, un polylactide, un polylactide-co-glycolide, des polyanhydrides, des poly-e-caprolactones, un polyméthacrylate de méthyle, un poly(N-isopropylacrylamide) ou des polypeptides.

3. Structure supramoléculaire pour son utilisation selon la revendication 2, dans laquelle ladite pluralité de composants structurels comprend un dendrimère.

4. Structure supramoléculaire pour son utilisation selon l'une quelconque des revendications 1, 2 ou 3, dans laquelle ladite pluralité de composants de terminaison comprend au moins l'un parmi le polyéthylène glycol, un dérivé d'adamantane, des ligands cibles, des peptides, des anticorps ou des protéines.

5. Structure supramoléculaire pour son utilisation selon l'une quelconque des revendications 1 et 2 à 4, dans laquelle la nanoparticule magnétique supramoléculaire (SMNP) a une taille prédéterminée d'au moins 30 nm et de moins de 500 nm.

6. Structure supramoléculaire pour son utilisation selon l'une quelconque des revendications 1 et 2 à 5, dans laquelle les régions de liaison se lient aux composants de terminaison ou aux composants structurels pour former une paire de reconnaissance moléculaire sélectionnée dans le groupe constitué par anticorps-antigène ; protéine-substrat ; protéine-inhibiteur ; protéine-protéine ; une paire d'oligonucléotides complémentaires ; et un complexe d'inclusion.

7. Structure supramoléculaire pour son utilisation selon la revendication 6, dans laquelle la paire de reconnaissance moléculaire est un complexe d'inclusion, de préférence adamantane-β-cyclodextrine ou diazobenzène-α-cyclodextrine.

8. Structure supramoléculaire pour son utilisation selon l'une quelconque des revendications 1 et 2 à 7, dans laquelle au moins l'un parmi le composant structurel, le composant de liaison, la nanoparticule magnétique ou le composant de terminaison comprend en outre un élément fonctionnel.

9. Structure supramoléculaire pour son utilisation selon la revendication 8, dans laquelle l'élément fonctionnel est un ligand de ciblage ou un ligand de perméation cellulaire.

10. Structure supramoléculaire pour son utilisation selon l'une quelconque des revendications 1 et 2 à 9, dans laquelle une cargaison supplémentaire est un ARNsi ; un peptide ; un oligonucléotide ; ou un plasmide.

11. Structure supramoléculaire pour son utilisation selon l'une quelconque des revendications 1 à 10, dans laquelle l'AMF est sélectionné pour accroître une température locale à proximité des nanoparticules magnétiques.

12. Structure supramoléculaire pour son utilisation selon l'une quelconque des revendications 1 à 11, dans laquelle la cargaison est substantiellement retenue par la structure de la SMNP préalablement à l'application de l'AMF.

13. Structure supramoléculaire pour son utilisation selon l'une quelconque des revendications 1 à 12, dans laquelle la SMNP comprend en outre un élément fonctionnel sélectionné parmi un ligand de ciblage et un ligand de perméation cellulaire.

14. Structure supramoléculaire pour son utilisation selon l'une quelconque des revendications 1 à 13, dans laquelle ledit procédé comprend en outre l'administration des SMNPs au sujet préalablement à la détermination de la distribution des SMNPs au sein de l'organisme du sujet.
